# EUROPEAN PATENT SPECIFICATION

(11) **EP 2 323 633 B1**
(45) Date of publication and mention of the grant of the patent: **21.03.2012**
(21) Application number: 09792207.4
(22) Date of filing: 03.09.2009
(51) Int. Cl.: A61K 9/20, A61K 31/00

(54) **STABLE PHARMACEUTICAL COMPOSITION FOR OPTIMIZED DELIVERY OF AN HIV ATTACHMENT INHIBITOR**
STABILE PHARMAZEUTISCHE ZUSAMMENSETZUNG ZUR OPTIMIERTEN ABGABE EINES HIV-ATTACHMENT-INHIBITORS
COMPOSITION PHARMACEUTIQUE STABLE POUR L'ADMINISTRATION OPTIMISÉE D'UN INHIBITEUR DE LA FIXATION DU VIH

(30) Priority: 04.09.2008 US 94131 P
(43) Date of publication of application: 25.05.2011
(73) Proprietor: Bristol-Myers Squibb Company, Princeton, NJ 08543-4000 (US)
(72) Inventor: BROWN, Jonathan, R., Merseyside CH46 1QW (GB); TOALE, Helen, Merseyside CH46 1QW (GB); DENNIS, Andrew, B., Merseyside CH46 1QW (GB); TIMMINS, Peter, Merseyside CH46 1QW (GB)
(74) Representative: Reitstötter - Kinzebach
(86) International application number: PCT/US2009/055820
(87) International publication number: WO 2010/028108

(56) References cited:
- WO-A2-2007/070589
- US-A1- 2005 209 246
- US-A1- 2006 293 304
- US-B2- 7 354 924
- WILLIAMS ROBERT O III ET AL: "INVESTIGATION OF EXCIPIENT TYPE AND LEVEL ON DRUG RELEASE FROM CONTROLLED RELEASE TABLETS CONTAINING HPMC" PHARMACEUTICAL DEVELOPMENT AND TECHNOLOGY, NEW YORK, NY, US LNKD- DOI:10.1081/PDT-120003486, vol. 7, no. 2, 1 January 2002 (2002-01-01) , pages 181-193, XP008074890 ISSN: 1083-7450

## Description

### FIELD OF THE INVENTION

The present invention relates to a tablet formulation providing an appropriate pharmacokinetic (PK) profile for the treatment of HIV infection, and more particularly, to a highly stable, extended release formulation containing an HIV attachment inhibitor and hydroxypropyl methylcellulose with no enzyme inhibitors that is efficacious against HIV.

### BACKGROUND OF THE INVENTION

HIV-1 (human immunodeficiency virus -1) infection remains a major medical problem, with close to 45 million people infected worldwide at the end of 2007. The number of cases of HIV and AIDS (acquired immunodeficiency syndrome) has risen rapidly. In 2005, for example, approximately 5.0 million new infections were reported, and 3.1 million people died from AIDS. Currently available drugs for the treatment of HIV include nucleoside reverse transcriptase (RT) inhibitors or approved single pill combinations: zidovudine (or AZT or Retrovir^{®}), didanosine (or Videx^{®}), stavudine (or Zerit^{®}), lamivudine (or 3TC or Epivir^{®}), zalcitabine (or DDC or Hivid^{®}), abacavir succinate (or Ziagen^{®}), Tenofovir disoproxil fumarate salt (or Viread^{®}), emtricitabine (or FTC or Emtriva^{®}), Combivir^{®} (contains -3TC plus AZT), Trizivir^{®} (contains abacavir, lamivudine, and zidovudine), Epzicom^{®} (contains abacavir and lamivudine), Truvada^{®} (contains Viread^{®} and Emtriva^{®}); non-nucleoside reverse transcriptase inhibitors: nevirapine (or Viramune^{®}), delavirdine (or Rescriptor^{®}) and efavirenz (or Sustiva^{®}), Atripla^{®} (Truvada^{®} + Sustiva^{®}), and etravirine, and peptidomimetic protease inhibitors or approved formulations: saquinavir, indinavir, ritonavir, nelfinavir, amprenavir, lopinavir, Kaletra^{®}(lopinavir and Ritonavir), darunavir, atazanavir (Reyataz^{®}), and tipranavir (Aptivus^{®}), and integrase inhibitors such as raltegravir (Isentress^{®}), and entry inhibitors such as enfuvirtide (T-20) (Fuzeon^{®}) and maraviroc (Selzentry^{®}).

In addition, HIV attachment inhibitors are a novel subclass of antiviral compounds that bind to the HIV surface glycoprotein gp120, and interfere with the interaction between the surface protein gp 120 and the host cell receptor CD4. Thus, they prevent HIV from attaching to the human CD4 T-cell, and block HIV replication in the first stage of the HIV life cycle. The properties of HIV attachment inhibitors have been improved in an effort to obtain compounds with maximized utility and efficacy as antiviral agents.

One HIV attachment inhibitor compound, in particular, has now shown considerable prowess against HIV. This compound is known as 1-benzoyl-4-[2-[4-methoxy-7-(3-methyl-1*H*-1,2,4-triazol-1-yl)-1-[(phosphonooxy)methyl]-1*H-*pyrrolo[2,3-c]pyridin-3-yl]-1,2-dioxoethyl]-piperazine. It is set forth and described in US 20050209246. The compound is represented by the formula below: 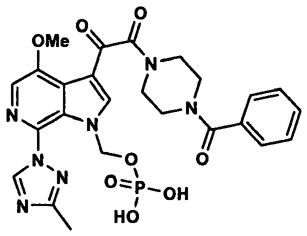 The above compound is the phosphate ester prodrug of the parent compound below, 1-(4-benzoyl-piperazin-1-yl)-2-[4-methoxy-7-(3-methyl-[1,2,4] triazol-1-yl)-1H-pyrralo [2,3-c] pyridine-3-yl]-ethane-1,2-dione, which is set forth and described in U.S. 7,354,924,

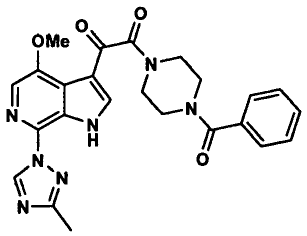

The phosphate ester prodrug is designed to be hydrolyzed by endogenous alkaline phosphatase to the parent compound immediately prior to absorption. Alkaline phosphatase is present in both a bound and an unbound state in the small and large intestine. If unbound enzyme in intestinal or colonic milieu is taken up into an extended release dosage form such as a matrix tablet, the enzyme could theoretically cause *in situ,* premature hydrolysis of the prodrug. If the prodrug is hydrolyzed within the tablet, this could prevent subsequent adequate absorption of the parent compound by the body. This, in turn, could mean increased dosing or worse, failed therapy.

What is therefore needed in the art is a dosage form containing the HIV attachment inhibitor prodrug compound 1-benzoyl-4-[2-[4-methoxy-7-(3-methyl-1*H-*1,2,4-triazol-1-yl)-1-[(phosphonooxy)methyl]-1*H*-pyrrolo[2,3-c]pyridin-3-yl]-1,2-dioxoethyl]-piperazine. This formulation should be stable and suitable for extended release, and desirably contain no enzyme inhibitors, and still protect the prodrug compound from hydrolysis within the dosage form following oral administration. Also needed are new methods of treatment against HIV infection using a stable tabletted form of the prodrug compound identified above.

### SUMMARY OF THE INVENTION

The invention provides a pharmaceutical composition, a tabat containing the compound 1-benzoyl-4-[2-[4-methoxy-7-(3-methyl-1*H*-1,2,4-triazol-1-yl)-1-[(phosphonooxy) methyl]-1*H*-pyrrolo[2,3-c]pyridin-3-yl]-1,2-dioxoethyl]-piperazine, and hydroxypropyl methyl cellulose (HPMC) having a viscosity of at least about 100 cP, wherein the composition does not contain any enzyme inhibitors and such HMPC is present in such composition in an amount of 10-50% by weight.

The invention also provides a pharmaceutical composition containing the phosphate ester prodrug compound 1-benzoyl-4-[2-[4-methoxy-7-(3-methyl-1*H-*1,2,4-triazol-1-yl)-1-[(phosphonooxy) methyl]-1*H*-pyrrolo[2,3-*c*]pyridin-3-yl]-1,2-dioxoethyl]-piperazine, and hydroxypropyl methyl cellulose (HPMC) having a viscosity of at least about 100 cP, wherein the composition does not contain any enzyme inhibitors and protects the prodrug from premature conversion to the parent compound before and after administration.

In a further embodiment, there is provided a pharmaceutical composition consisting essentially of the compound 1-benzoyl-4-[2-[4-methoxy-7-(3-methyl-1*H-*1,2,4-triazol-1-yl)-1-[(phosphonooxy)methyl]-1*H*-pyrrolo[2,3-*c*]pyridin-3-yl]-1,2-dioxoethyl]-piperazine, HPMC having a viscosity of at least about 2000 cP, microcrystalline cellulose, silicon dioxide and magnesium stearate, wherein the composition does not contain any enzyme inhibitors.

Also provided herein is a pharmaceutical composition consisting essentially of the compound 1-benzoyl-4-[2-[4-methoxy-7-(3-methyl-1*H*-1,2,4-triazol-1-yl)-1-[(phosphonooxy)methyl]-1*H*-pyrrolo[2,3-*c*]pyridin-3-yl]-1,2-dioxoethyl]-piperazine, HPMC having a viscosity of at least about 3000 cP, microcrystalline cellulose, silicon dioxide and magnesium stearate, wherein the composition does not contain any enzyme inhibitor, and further wherein the compound within the composition is substantially resistant to hydrolysis by the enzyme alkaline phosphatase.

Further provided is a method for treating a mammal infected with the HIV virus comprising administering to said mammal an antiviral effective amount of one or more of the compositions set forth above.

In addition, there is set forth a method for preparing a pharmaceutical composition, which comprises admixing the compound 1-benzoyl-4-[2-[4-methoxy-7-(3-methyl-1*H*-1,2,4-triazol-1-yl)-1-[(phosphonooxy)methyl]-1*H*-pyrrolo[2,3-*c*]pyridin-3-yl]-1,2-dioxoethyl]-piperazine together with HPMC having a viscosity of at least about 2000 cP in the absence of any enzyme inhibitors.

The present invention is directed to these, as well as other important ends, hereinafter described.

The composition of the invention according to its various embodiments will permit extended release of the HIV attachment inhibitor prodrug both *in vitro* and *in vivo.* Furthermore, the composition of the invention will protect the HIV attachment inhibitor prodrug within the dosage form from enzymatic hydrolysis. The resultant *in vivo* release profile will lead to an optimized plasma-time profile. The mechanism of protection appears to be complex and related to the formulation as a whole, and not just to one of the excipients.

### DETAILED DESCRIPTION OF THE EMBODIMENTS

The HIV attachment inhibitor compound known as 1-benzoyl-4-[2-[4-methoxy-7-(3-methyl-1*H*-1,2,4-triazol-1-yl)-1-[(phosphonooxy)methyl]-1*H-*pyrrolo[2,3-*c*]pyridin-3-yl]-1,2-dioxoethyl]-piperazine is a phosphate ester prodrug as set forth below: 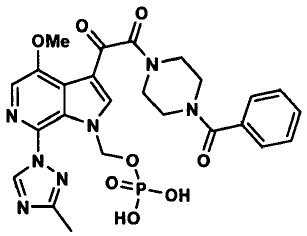 It is designed to be hydrolyzed by endogenous alkaline phosphatase to the parent compound below immediately prior to absorption: 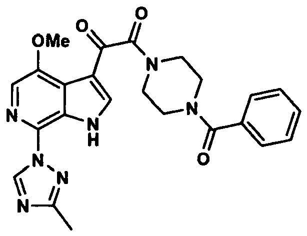 The phosphate ester prodrug has now been shown to rapidly hydrolyze to the parent compound when alkaline phosphatase is present in solution. In order to reduce the frequency of dosing, 1-benzoyl-4-[2-[4-methoxy-7-(3-methyl-1*H*-1,2,4-triazol-1-yl)-1-[(phosphonooxy)methyl]-1*H*-pyrrolo[2,3-*c*]pyridin-3-yl]-1,2-dioxoethyl]-piperazine has now been formulated into a stable, hydrophilic matrix extended release tablet. The tablet formulation has been shown to swell and form a gel when exposed to aqueous media. The phosphate ester prodrug compound 1-benzoyl-4-[2-[4-methoxy-7-(3-methyl-1*H*-1,2,4-triazol-1-yl)-1-[(phosphonooxy)methyl]-1*H-*pyrrolo[2,3-*c*]pyridin-3-yl]-1,2-dioxoethyl]-piperazine is then released from the hydrated tablet into solution via diffusional and/or erosional processes over an extended period.

It is now also been shown that the hydrophilic matrix of the tablet protects the phosphate ester prodrug from *in situ* hydrolysis to the parent compound within the hydrated tablet. This permits the release of the prodrug to occur over an extended time period leading to a desired pharmacokinetic profile without the need to incorporate enzyme inhibitors into the formulation. If the prodrug were to be hydrolyzed within the tablet, the resulting parent compound would be unavailable for absorption and the desired pharmacokinetic profile would not be achieved.

Thus, the composition of the invention provides an extended release formulation containing 1-benzoyl-4-[2-[4-methoxy-7-(3-methyl-1*H*-1,2,4-triazol-1-yl)-1-[(phosphonooxy)methyl]-1*H*-pyrrolo[2,3-*c*]pyridin-3-yl]-1,2-dioxoethyl]-piperazine. The formulation contains about 20 - 90% by weight of the compound. In a more preferred embodiment, there is provided about 50 - 85% by weight of the compound. Even more preferably, the formulation contains about 60 - 75% by weight of the prodrug ester compound.

While a range of alternative water soluble salts of the phosphate ester prodrug may be employed, the tris salt form of 1-benzoyl-4-[2-[4-methoxy-7-(3-methyl-1*H-*1,2,4-triazol-1-yl)-1-[(phosphonooxy)methyl]-1*H*-pyrrolo[2,3-*c*]pyridin-3-yl]-1,2-dioxoethyl]-piperazine, in particular, is preferred for use herein.

Also included in the formulation is hydroxypropyl methyl cellulose (HPMC). HPMC is a pharmaceutical grade polymer that is utilized in forming extended release formulations. The HPMC is present in the composition in an amount of about 10-50% by weight thereof. More preferably, the amount of HPMC is about 15-40% by weight. Even more desirably, the composition contains about 20-30% by weight of HPMC.

It is preferred that the HPMC utilized as part of the composition of the invention have a relatively high hydrated viscosity. Preferably, the viscosity of a 2% solution of HPMC in water should be at least about 100 centipoise (cP). Even more preferably, the viscosity should be at least about 2000 cP. More desirably, the viscosity of the HPMC which is included in the formulation should be at least about 3000 cP. In some embodiments, it is even more preferred that the HPMC have a viscosity of at least about 4000 cP. It is also preferred that the viscosity of the HPMC not exceed about 10,000 cP, although in some applications, an upper limit viscosity of about 50,000 cP, or even about 120,000 cP may be suitable.

It is also preferable that the HPMC used as part of the invention have a methoxy substitution which is substantially higher than the degree of hydroxypropoxy substitution on the molecule. For example, HPMC having about 22% methoxy substitution and about 8% hydroxypropoxy substitution is highly preferred for use herein.

In addition to the foregoing components, including the HPMC, the composition of the invention contains one or more additional excipients. These excipients are desirably chosen to aid in the development of the final formulation matrix. Excipients include fillers, flow aids, glidants, lubricants, binders, diluents, disintegrants, preservatives and the like. These may be selected from the group consisting of microcrystalline cellulose, silicon dioxide, and magnesium stearate. Pharmaceutical grade film-coating materials may also be utilized. Other excipients available to the skilled artisan may also be utilized herein. These additional excipient(s) are present in the formulation in an amount of about 0.01 to 15% by weight thereof. More preferably, the quantity of additional excipient(s) is within the range of about 0.5 to 10% by weight of the final formulation.

In a further embodiment wherein a tablet potency of lower than about 600 mg is required, a quantity of lactose or other soluble excipients may be included in the formulation in place of prodrug.

As set above, the formulation does not contain any enzyme inhibitors. It has now been surprisingly shown that the prodrug compound, normally highly susceptible to hydrolysis by the enzyme alkaline phosphatase, is stable in the final formulation and is not hydrolyzed in situ within the tablet matrix. Instead, the compound can be suitably released from the tablet over an extended time period for adequate conversion to the parent compound, and subsequent absorption by the body. This conversion to the parent compound takes place just prior to absorption only, and not within the dosage form itself.

The composition herein described and set forth may be prepared by mixing the prodrug compound 1-benzoyl-4-[2-[4-methoxy-7-(3-methyl-1*H*-1,2,4-triazol-1-yl)-1-[(phosphonooxy)methyl]-1*H*-pyrrolo[2,3-*c*]pyridin-3-yl]-1,2-dioxoethyl]-piperazine together with the additional excipient(s) according to methods available in the art. Thereafter, the HPMC may be combined with the other components, and the final formulation may be compressed into tablets using a tablet press. In an alternative embodiment, the HPMC is mixed together at the same time with the prodrug and additional excipients. Film-coating materials may also be applied onto the surface of the tablet so formed, if desired.

The tabletted formulation is highly storage stable, and also allows the prodrug ester to be reliably released from the tablet matrix over an extended period in the gut, preferably over the period from about 4 to 24 hours, more preferably about 8 to 24 hours, and even more preferably, about 12 to 24 hours.

As set forth above, the prodrug compound in the composition herein set forth is substantially resistant to hydrolysis by the enzyme alkaline phosphatase. This means that there is less than about 1% conversion of the prodrug compound within the tablet matrix after about 5 hours exposure to the amount of alkaline phosphatase enzyme (from porcine intestine) at a concentration of about 1 mg/mL.

The composition of the invention can be administered orally to humans in a dosage range of about 1 to 100 mg/kg body weight in divided doses, normally over an extended period of days, weeks, months or even years. One preferred dosage range is about 1 to 20 mg/kg body weight orally in singular or divided doses daily. Another preferred dosage range is about 1 to 40 mg/kg body weight in singular or divided doses daily. Yet another preferred dosage range is from about 600 - 1200 mg total twice a day. It will be understood, however, that the specific dose level and frequency of dosage for any particular patient may be varied and will depend upon a variety of factors including the activity of the compound, the metabolic stability and length of action of that compound, the age, body weight, general health, sex, diet, mode and time of administration, rate of excretion, drug combination, the severity of the particular condition, and the host undergoing therapy.

Also contemplated herein are combinations of the formulation herein set forth, according to its various embodiments, together with one or more other agents useful in the treatment of AIDS. For example, the compositions of this disclosure may be effectively administered, whether at periods of pre-exposure and/or post-exposure, in combination with effective amounts of the AIDS antivirals, immunomodulators, antiinfectives, or vaccines available in the art, including those set forth herein in the Background of the Invention.

The following examples illustrate one or preferred aspects of the invention,

### Example 1: A 600 mg Extended Release Tablet Formulation

| Ingredient | Function | mg per tablet |
|---|---|---|
| Prodrug | API | 725* |
| HPMC (4000cP) | Extended Release Polymer | 283 |
| Microcrystalline cellulose | Compression aid | 95 |
| Silicon dioxide | Glidant | 17 |
| Magnesium stearate | Lubricant | 10 |

| | | |
|---|---|---|
| *Tris salt equivalent to 600 mg free acid | | |

### Example 2: An Alternative 600 mg Extended Release Tablet Formulation

| Ingredient | Function | mg per tablet |
|---|---|---|
| Prodrug | API | 725* |
| HPMC (4000cP) | Extended Release Polymer | 85 |
| HPMC (100 cP) | Extended Release Polymer | 198 |
| Microcrystalline cellulose | Compression aid/Filler | 95 |
| Silicon dioxide | Glidant | 17 |
| Magnesium stearate | Lubricant | 10 |

| | | |
|---|---|---|
| *Tris salt equivalent to 600 mg free acid | | |

### Example 3: An Alternative 600 mg Extended Release Tablet Formulation

| Ingredient | Function | mg per tablet |
|---|---|---|
| Prodrug | API | 725* |
| HPMC (4000cP) | Extended Release Polymer | 28 |
| HPMC (100 cP) | Extended Release Polymer | 255 |
| Microcrystalline cellulose | Compression aid/Filler | 101 |
| Silicon dioxide | Glidant | 13 |
| Magnesium stearate | Lubricant | 10 |

| | | |
|---|---|---|
| *Tris salt equivalent to 600 mg free acid | | |

### Example 4: An Alternative 600 mg Extended Release Tablet Formulation

| Ingredient | Function | mg per tablet |
|---|---|---|
| Prodrug | API | 725* |
| HPMC (4000cP) | Extended Release Polymer | 283 |
| Microcrystalline cellulose | Compression aid/Filler | 103 |
| Silicon dioxide | Glidant | 9 |
| Magnesium stearate | Lubricant | 10 |

| | | |
|---|---|---|
| *Tris salt equivalent to 600 mg free acid | | |

### Example 5: A 200 mg Extended Release Formulation

| Ingredient | Function | mg per tablet |
|---|---|---|
| Prodrug | API | 242* |
| HPMC (4000cP) | Extended Release Polymer | 283 |
| Microcrystalline cellulose | Compression aid/filler | 34 |
| Lactose | Filler | 562 |
| Silicon dioxide | Glidant | 4 |
| Magnesium stearate | Lubricant | 7 |

| | | |
|---|---|---|
| *Tris salt equivalent to 200 mg free acid | | |

### Example 6: A 600 mg Extended Release Tablet Formulation

| Ingredient | Function | mg per tablet |
|---|---|---|
| Prodrug | API | 725* |
| HPMC (4000cP) | Extended Release Polymer | 283 |
| Microcrystalline cellulose | Compression aid | 95 |
| Silicon dioxide | Glidant | 17 |
| Magnesium stearate | Lubricant | 10 |
| Opadry II 85F | Film coat | 34 |

| | | |
|---|---|---|
| *Tris salt equivalent to 600 mg free acid | | |

### Example 7: In vitro Drug Release Profiles for Example Extended Release Tablet Formulations Using USP Type 1 Apparatus With Baskets Rotating at 100 rpm and a pH 6.8 Phosphate buffer.

Tablets from Examples 1 to 6 permit release of drug over 10 to 24 hours. This slow release is designed to enable plasma concentrations of the parent compound to be maintained at a therapeutic level for a prolonged period of time. The release profile for the immediate release capsule is included for illustrative purposes.

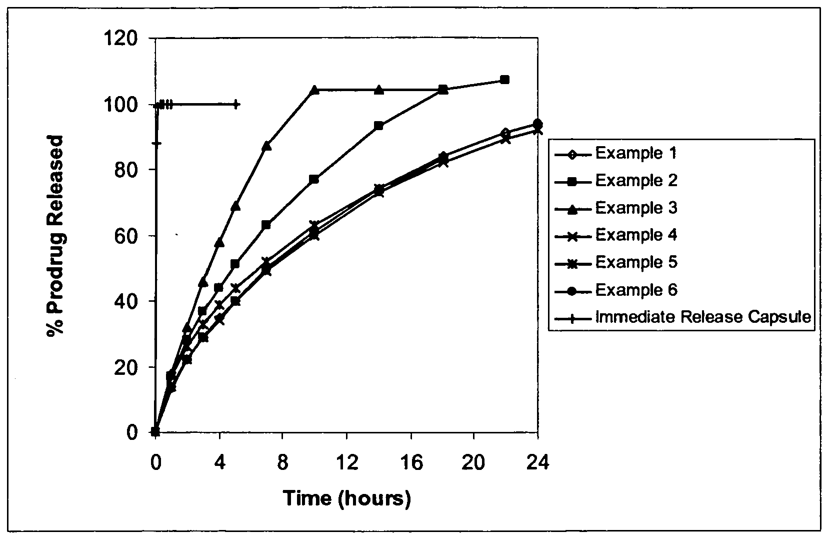

### Example 8: Tablets from Example 1 Administered as a Single Dose to Human Volunteers Resulting in a Desired Plasma Concentration - Time Profile.

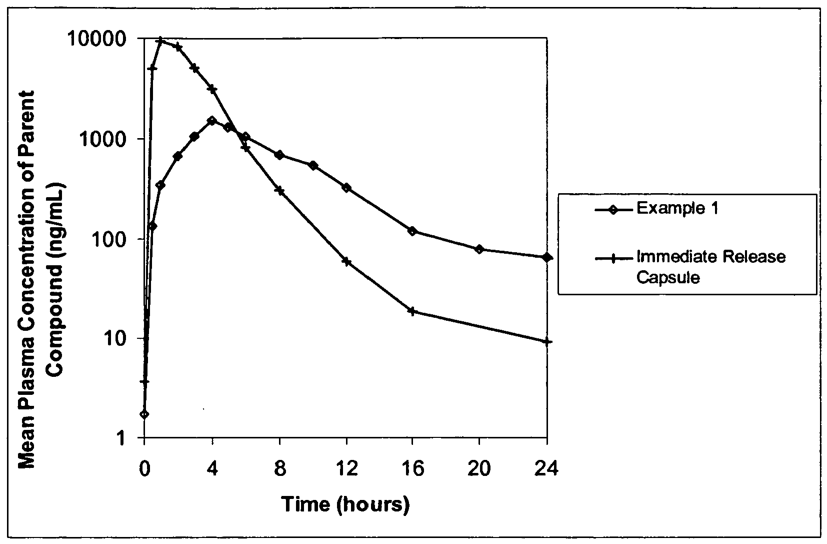

The extended release tablets maintain parent compound plasma concentrations at therapeutic levels throughout the 12 hour dosing period. This is not achieved via dosing of the immediate release capsule (profile shown above for illustrative purposes). Furthermore, the extended release tablets deliver a lower peak concentration (Cmax) compared to the immediate release capsules. This is desirable to minimize Cmax related adverse events.

### Example 9: Alkaline phosphatase is shown to rapidly convert prodrug to parent compound in pH 7.0 Tris-HCl buffer.

Phosphate ester prodrug was dissolved in pH 7.0 Tris-HCl buffer to a concentration of 0.72 mg/mL. Porcine alkaline phosphatase (1 mg/mL) was added to an aliquot of the solution. 2.5 hours after enzyme addition a sample was analyzed. The results in the Table below confirm that alkaline phosphatase is active in pH 7.0 Tris-HCl buffer and rapidly hydrolyzes the phosphate ester prodrug to the parent compound.

| Sample Description | Prodrug peak area* | Parent peak area* |
|---|---|---|
| Initial sample of prodrug solution (no enzyme) | 99.9% | 0.1% |
| 2.5 hr sample of solution containing enzyme | 0.0% | 100.0% |

| | | |
|---|---|---|
| * expressed as percent relative to total peak area for prodrug and parent | | |

### Example 10: Alkaline phosphatase is shown to permeate and remain biologically active in a hydrated HPMC gel matrix

To determine whether the hydrated HPMC polymer acts as a barrier to entry of enzyme into the gel layer, HPMC discs containing an enzyme substrate were prepared. Compacts of HPMC (viscosity 4000 cP) containing 5-bromo-4-chloro-3-indoyl disodium, BCIP (enzyme substrate) and nitrotetrazolium blue chloride, NBT (color reagent) were hydrated in pH 7.0 Tris-HCl buffer containing 1 mg/mL alkaline phosphatase. A purple precipitate was observed within the gel after 3 hours, confirming activity of the enzyme against the BCIP substrate in the compact. This confirms that alkaline phosphatase is able to enter the HPMC gel from solution and is biologically active therein. The HPMC does not therefore act as a diffusion barrier to the drug.

### Example 11: The Stability of the Phosphate Ester Prodrug is Demonstrated Within Hydrating Tablets of Example 3 in the Presence of Alkaline Phosphatase.

Tablets of Example 3 were used in dissolution tests with pH 7.0 Tris-HCl buffer containing porcine alkaline phosphatase (1 mg/mL). USP Type 1 apparatus was employed with baskets rotating at 100 rpm. At 2.5 hours and 4.5 hours after the start of the experiment, tablets were removed from the buffer and subjected to grinding and solvent extraction to analyze the prodrug and parent compounds. Samples of the dissolution medium were also analyzed.

The table below shows the HPLC peak areas associated with prodrug and parent compound from tablet and buffer solution samples. The high ratio of parent peak area to prodrug peak area in buffer solution samples confirms the rapid conversion of the prodrug to parent compound within the enzyme containing solution. The very low ratio of parent peak area to prodrug peak area following extraction from the tablet indicates that no substantial conversion of the prodrug to the parent compound has occurred within the hydrating tablet over the duration of the experiment. This confirms the protection of the prodrug from alkaline phosphatase-mediated conversion within the hydrated tablet.

| Sample Description | Prodrug peak area* | Parent peak area* |
|---|---|---|
| 2.5 hr sample of dissolution medium | 1.3% | 98.7 % |
| 2.5 hr tablet extraction | 99.8% | 0.2% |
| 4.5 hr sample of dissolution medium | 1.1 % | 98.9 % |
| 4.5 hr tablet extraction | 99.7% | 0.3% |

| | | |
|---|---|---|
| * expressed as percent relative to total peak area for prodrug and parent | | |

### Example 12: The Stability of the Phosphate Ester Prodrug is Demonstrated Within Hydrating Tablets of Example 4 in the Presence of Alkaline Phosphatase.

Tablets of Example 4 were used in dissolution tests with pH 7.0 Tris-HCl buffer containing porcine alkaline phosphatase (1 mg/mL) as described in Example 11. Tablets and samples of dissolution medium were removed at 3 hours and 7 hours after the start of the experiment.

The table below shows the HPLC peak areas associated with prodrug and parent compound from tablet and buffer solution samples. As in Example 11, rapid conversion of the prodrug to parent was observed within the dissolution medium and no substantial conversion of the prodrug to the parent was observed within the hydrating tablet. This confirms the protection of the prodrug from alkaline phosphatase within formulations containing different viscosity grades of HPMC and therefore hydrated gel layers of different viscosities.

| Sample Description | Prodrug peak area* | Parent peak area* |
|---|---|---|
| 3 hr sample of dissolution medium | 8.3% | 91.7% |
| 3 hr tablet extraction | 99.6% | 0.4% |
| 7 hr sample of dissolution medium | 6.5% | 93.5 % |
| 7 hr tablet extraction | 98.3% | 1.7% |

| | | |
|---|---|---|
| * expressed as percent relative to total peak area for prodrug and parent | | |

### Example 13: The Stability of the Phosphate Ester Prodrug in Example 4 is Shown to be Independent of Local pH Effects

In vitro drug release experiments performed in pH 7.0 Tris-HCl buffer containing 0.5% Universal indicator show the hydrated gel layer of Example 4 tablets to be acidic (approximately pH 3). To confirm whether the acidic pH inhibits the enzyme, Basified Example 4 tablets were created by substituting the acidic prodrug with basic Tris within the tablet formula as shown below. When hydrated in pH 7.0 Tris-HCl buffer, these tablets were shown to have a weakly basic gel layer (approximately pH 8). This pH is expected to be within the acceptable range for enzyme activity.

| Ingredient | Function | mg per tablet | |
|---|---|---|---|
| | | Basified Example 4 | Un-modified Example 4 |
| Prodrug | API | 591* | 725** |
| Tris | Base | 136 | 0 |
| HPMC (4000cP) | Extended Release Polymer | 283 | 283 |
| Microcrystalline cellulose | Compression aid/Filler | 101 | 103 |
| Silicon dioxide | Glidant | 9 | 9 |
| Magnesium stearate | Lubricant | 10 | 10 |

| | | | |
|---|---|---|---|
| * Tris salt equivalent to 489 mg of free acid ** Tris salt equivalent to 600 mg of free acid | | | |

Basified Example 4 tablets were used in dissolution tests with pH 7.0 Tris-HCl buffer containing porcine alkaline phosphatase (1 mg/mL) as described in Example 12. Tablets and samples of dissolution medium were removed at 3 hours and 7 hours after the start of the experiment. and analyzed.

The results below show no substantial conversion of the prodrug to the parent occurring within the basified tablets. This confirms that the prodrug stabilization within the gel layer was not modulated by pH as would have been expected.

| Sample Description | Prodrug peak area* | Parent peak area* |
|---|---|---|
| 3 hr sample of dissolution medium | 2.0% | 98.0 % |
| 3 hr tablet extraction | 99.0% | 1.0% |
| 7 hr sample of dissolution medium | 1.1 % | 98.9 % |
| 7 hr tablet extraction | 98.0% | 2.0% |

| | | |
|---|---|---|
| * expressed as percent relative to total peak area for prodrug and parent | | |

### Example 14: Alkaline Phosphatase is Shown to be Active in a Formulation Where the Prodrug has been Replaced by the Parent Compound

To investigate whether inhibition of enzyme activity is observed in tablets containing drug substances other than the prodrug, compacts weighing 200 mg were prepared containing parent drug and the enzyme substrate, BCIP/NBT, in an extended release formulation. BCIP/NBT was included to detect alkaline phosphatase activity in the formulations. Compacts containing prodrug were also prepared for comparison. In the prodrug formulation, Tris is included therein to adjust the pH to within an acceptable range for enzyme activity (approx.pH 8). This is not necessary in the parent drug formulation, as pH is already expected to be within the acceptable range. The enzyme activity demonstrated in Tris-HCl buffer (ref. Example 9) strongly suggests that Tris itself is not an enzyme inhibitor, and would not be expected to have a direct impact on enzyme activity.

| Ingredient | Function | mg per compact | |
|---|---|---|---|
| | | Parent formulation | Prodrug formulation |
| Prodrug | API | - | 105.9 |
| Parent | API | 85.9 | - |
| Tris | Base | - | 24.0 |
| HPMC (4000cP) | Extended Release Polymer | 49.9 | 49.9 |
| Microcrystalline cellulose | Compression aid/Filler | 60.2 | 16.2 |
| Silicon dioxide | Glidant | 1.6 | 1.6 |
| Magnesium stearate | Lubricant | 1.8 | 1.8 |
| BCIP | Enzyme substrate | 0.2 | 0.2 |
| NBT | Color product | 0.4 | 0.4 |

Compacts were hydrated in pH 7.0 Tris-HCl buffer containing 1 mg/mL porcine alkaline phosphatase. Following 3 hours hydration, a strong blue/purple coloration throughout the gel was observed in the compacts containing parent molecule indicating a high level of enzyme activity therein. By comparison very slight coloration was observed in compacts containing prodrug indicating very low level enzyme activity. These results confirm that alkaline phosphatase is active in the formulation containing the parent drug and not in the formulation containing prodrug.

When considered together, the results in Examples 9 to 14 imply a complex system in which an unexpected inhibition of alkaline phosphatase activity occurs within an extended release formulation containing the HIV attachment produg molecule.

## Claims

1. A pharmaceutical composition in the form of a tablet comprising the phosphate ester prodrug of an HIV attachment inhibitor, 1-benzoyl-4-[2-[4-methoxy-7-(3-methyl-1H-1,2,4-triazol-1-yl)-1-[(phosphonooxy)methyl]-1H-pyrrolo[2,3-*c*]pyridin-3-yl]-1,2-dioxoethyl]-piperazine, and hydroxypropyl methyl cellulose (HPMC) having a hydrated viscosity of at least about 100 cP, that provides a desired extended absorption of the parent compound when administered to humans, provides for stability of the prodrug against alkaline phosphatase while still contained within the dosage form under post-administration conditions, and wherein said composition does not contain any enzyme inhibitors and said HPMC is present is said composition in an amount of 10-50% by weight.

2. The composition of claim 1, wherein said compound is present in said composition in an amount of 20 - 90% by weight.

3. The composition of claim 2, wherein said compound is present in an amount of 50-85% by weight.

4. The composition of claim 3, wherein said compound is present in an amount of 60-75% by weight.

5. The composition of claim 1, wherein said HPMC is present in an amount of 15-40% by weight.

6. The composition of claim 5, wherein said HPMC is present in an amount of 20-30% by weight.

7. The composition of claim 1, wherein said HPMC has a viscosity of at least about 2000 cP, at least about 3000 cP or at least about 4000 cP.

8. The composition of claim 1, further comprising one or more additional excipients selected from the group consisting of microcrystalline cellulose, silicon dioxide, and magnesium stearate.

9. The composition of claim 8, wherein said additional excipients are present in an amount of 15% by weight of said composition.

10. The composition of claim 1, wherein said viscosity of the HMPC does not exceed about 120,000 cP.

11. The composition of claim 1, wherein said compound is in the form of a tris salt.

12. The composition of claim 1, useful for treating infection by HIV, which additionally comprises an antiviral effective amount of an AIDS treatment agent selected from the group consisting of:
(a) an AIDS antiviral agent;
(b) an anti-infective agent;
(c) an immunomodulator; and
(d) another HIV entry inhibitor.

13. A method for preparing a pharmaceutical composition in the form of a tablet, which comprises admixing the compound 1-benzoyl-4-[2-[4-methoxy-7-(3-methyl-1H-1,2,4-triazol-1-yl)-1-[(phosphonooxy)methyl]-1H-pyrrolo[2,3-*c*]pyridin-3-yl]-1,2-dioxoethyl]-piperazine together with HPMC having a viscosity of at least about 2000 cP in the absence of any enzyme inhibitors and compressing said composition into a tablet.

14. A composition as claimed in any one of claims 1 to 12 for use in treating a mammal infected with the HIV virus.

15. The composition for use according to claim 14, comprising administering to said mammal an antiviral effective amount of said composition, in combination with an antiviral effective amount of an AIDS treatment agent selected from the group consisting of an AIDS antiviral agent; an anti-infective agent; an immunomodulator; and another HIV entry inhibitor.

## Patentansprüche

1. Pharmazeutische Zusammensetzung in Form einer Tablette, die das Phosphatester-Prodrug eines HIV-Attachment-Inhibitors, 1-Benzoyl-4-[2-[4-methoxy-7-(3-methyl-1H-1,2,4-triazol-1-yl)-1-[(phosphonoxy)methyl]-1H-pyrrol[2,3-c]pyridin-3-yl]-1,2-dioxoethyl]-piperazin, und Hydroxypropylmethylcellulose (HPMC) mit einer Viskosität im hydratisierten Zustand von mindestens etwa 100 cP aufweist, die eine gewünschte Langzeitaufnahme der Stammverbindung bei Verabreichung an Menschen sichert, für Stabilität des Prodrugs gegen alkalische Phosphatase sorgt und dabei unter nach der Verabreichung herrschenden Bedingungen noch in der Dosierungsform enthalten ist, wobei die Zusammensetzung keine Enzyminhibitoren enthält und die HPMC in einem Anteil von 10-50 Gew.-% in der Zusammensetzung enthalten ist.

2. Zusammensetzung nach Anspruch 1, wobei die Verbindung in einem Anteil von 20-90 Gew.-% in der Zusammensetzung enthalten ist.

3. Zusammensetzung nach Anspruch 2, wobei die Verbindung in einem Anteil von 50-85 Gew.-% enthalten ist.

4. Zusammensetzung nach Anspruch 3, wobei die Verbindung in einem Anteil von 60-75 Gew.-% enthalten ist.

5. Zusammensetzung nach Anspruch 1, wobei die HPMC in einem Anteil von 15-40 Gew.-% enthalten ist.

6. Zusammensetzung nach Anspruch 5, wobei die HPMC in einem Anteil von 20-30 Gew.-% enthalten ist.

7. Zusammensetzung nach Anspruch 1, wobei die HPMC eine Viskosität von mindestens etwa 2000 cP, mindestens etwa 3000 cP oder mindestens etwa 4000 cP aufweist.

8. Zusammensetzung nach Anspruch 1, die ferner einen oder mehrere zusätzliche Trägerstoffe aufweist, die aus der Gruppe ausgewählt sind, die aus mikrokristalliner Cellulose, Siliciumdioxid und Magnesiumstearat besteht.

9. Zusammensetzung nach Anspruch 8, wobei die zusätzlichen Trägerstoffe in einem Anteil von 5-15 Gew.-% der Zusammensetzung enthalten sind.

10. Zusammensetzung nach Anspruch 1, wobei die Viskosität der HPMC 120000cP nicht übersteigt.

11. Zusammensetzung nach Anspruch 1, wobei die Verbindung in Form eines Tris-Salzes vorliegt.

12. Zusammensetzung nach Anspruch 1, die für die Behandlung einer Infektion durch HIV einsetzbar ist und außerdem eine antiviral wirksame Menge eines Wirkstoffs zur AIDS-Behandlung aufweist, der aus der Gruppe ausgewählt ist, die aus den folgenden Wirkstoffen besteht:
(a) einem AIDS-Virostatikum;
(b) einem Antiinfektionsmittel;
(c) einem Immunmodulator; und
(d) einem weiteren HIV-Eintrittsinhibitor.

13. Verfahren zur Herstellung einer pharmazeutischen Zusammensetzung in Form einer Tablette, wobei das Verfahren aufweist: Vermischen der Verbindung 1-Benzoyl-4-[2-[4-methoxy-7-(3-methyl-1H-1,2,4-triazol-1-yl)-1-[(phosphonoxy)methyl]-1H-pyrrol[2,3-c]pyridin-3-yl]-1,2-dioxoethyl]-piperazin mit HPMC, die eine Viskosität von mindestens etwa 2000 cP aufweist, in Abwesenheit von Enzyminhibitoren und Pressen der Zusammensetzung zu einer Tablette.

14. Zusammensetzung nach einem der Ansprüche 1 bis 12 zur Verwendung bei der Behandlung eines mit dem HIV-Virus infizierten Säugetiers.

15. Zusammensetzung zur Verwendung gemäß Anspruch 14, wobei eine antiviral wirksame Menge der Zusammensetzung in Kombination mit einer antiviral wirksamen Menge eines Wirkstoffs zur Behandlung von AIDS, der aus der Gruppe ausgewählt ist, die aus einem AIDS-Virostatikum, einem Antiinfektionsmittel, einem Immunmodulator und einem weiteren HIV-Eintrittsinhibitor besteht, an das Säugetier verabreicht wird.

## Revendications

1. Composition pharmaceutique sous forme de comprimé comprenant le promédicament ester de phosphate d'un inhibiteur de fixation du VIH, la 1-benzoyl-4-[2-[4-méthoxy-7-(3-méthyl-1H-1,2,4-triazol-1-yl)-1-[(phosphonooxy)méthyl]-1H-pyrrolo[2,3-c]pyridin-3-yl]-1,2-dioxoéthyl]-pipérazine, et de l'hydroxypropylméthylcellulose (HPMC) ayant une viscosité à l'état hydraté d'au moins environ 100 cP, qui assure une absorption prolongée voulue du composé parent en cas d'administration à des sujets humains, et permet la stabilité du promédicament vis-à-vis de la phosphatase alcaline alors que celui-ci est toujours contenu à l'intérieur de la forme pharmaceutique dans des conditions de post-administration, ladite composition ne contenant pas d'inhibiteurs d'enzyme et ladite HPMC étant présente dans ladite composition en une quantité de 10-50% en poids.

2. Composition selon la revendication 1, dans laquelle ledit composé est présent dans ladite composition en une quantité de 20-90% en poids.

3. Composition selon la revendication 2, dans laquelle ledit composé est présent en une quantité de 50-85% en poids.

4. Composition selon la revendication 3, dans laquelle ledit composé est présent en une quantité de 60-75% en poids.

5. Composition selon la revendication 1, dans laquelle ladite HPMC est présente en une quantité de 15-40% en poids.

6. Composition selon la revendication 5, dans laquelle ladite HPMC est présente en une quantité de 20-30% en poids.

7. Composition selon la revendication 1, dans laquelle ladite HPMC a une viscosité d'au moins environ 2000 cP, d'au moins environ 3000 cP ou d'au moins environ 4000 cP.

8. Composition selon la revendication 1, comprenant en outre un ou plusieurs excipients supplémentaires choisis dans le groupe constitué de la cellulose microcristalline, du dioxyde de silicium, et du stéarate de magnésium.

9. Composition selon la revendication 8, dans laquelle lesdits excipients supplémentaires sont présents en une quantité de 5-15% en poids de ladite composition.

10. Composition selon la revendication 1, dans laquelle ladite viscosité de l'HPMC ne dépasse pas environ 120000 cP.

11. Composition selon la revendication 1, dans laquelle ledit composé se présente sous la forme d'un sel de tris.

12. Composition selon la revendication 1, utile pour traiter une infection par le VIH, qui comprend en outre une quantité antivirale efficace d'un agent de traitement du SIDA choisi dans le groupe constitué de:
(a) un agent antiviral contre le SIDA;
(b) un agent anti-infectieux;
(c) un immunomodulateur; et
(d) un autre inhibiteur d'entrée du VIH.

13. Procédé de préparation d'une composition pharmaceutique sous forme de comprimé, qui comprend les étapes consistant à mélanger le composé 1-benzoyl-4-[2-[4-méthoxy-7-(3-méthyl-1H-1,2,4-triazol-1-yl)-1-[(phosphonooxy)méthyl]-1H-pyrrolo[2,3-c]pyridin-3-yl]-1,2-dioxoéthyl]-pipérazine avec de l'HPMC ayant une viscosité d'au moins environ 2000 cP en l'absence de tout inhibiteur d'enzyme, et à compresser ladite composition pour en faire un comprimé.

14. Composition selon l'une quelconque des revendications 1 à 12 destinée à être utilisée dans le traitement d'un mammifère infecté par le virus VIH.

15. Composition pour utilisation selon la revendication 14, comprenant l'administration audit mammifère d'une quantité antivirale efficace de ladite composition, en combinaison avec une quantité antivirale efficace d'un agent de traitement du SIDA choisi dans le groupe constitué d'un agent antiviral contre le SIDA; d'un agent anti-infectieux; d'un immunomodulateur; et d'un autre inhibiteur d'entrée du VIH.
